# EUROPEAN PATENT SPECIFICATION

(11) **EP 4 351 764 B1**
(45) Date of publication and mention of the grant of the patent: **27.08.2025**
(21) Application number: 21736046.0
(22) Date of filing: 09.06.2021
(51) Int. Cl.: B01D 53/62, B01D 53/96, C01B 3/08

(54) **SYSTEM AND METHOD FOR CARBON CAPTURE AND UTILIZATION**
SYSTEM UND VERFAHREN ZUR KOHLENSTOFFABSCHEIDUNG UND -VERWENDUNG
SYSTÈME ET PROCÉDÉ DE CAPTURE ET D'UTILISATION DE CARBONE

(43) Date of publication of application: 17.04.2024
(73) Proprietor: CYPRUS UNIVERSITY OF TECHNOLOGY, 3603 Lemesos (CY)
(72) Inventor: VYRIDES, Ioannis, 2021 Nicosia (CY)
(74) Representative: De Tullio, Michele Elio
(86) International application number: PCT/IB2021/055067
(87) International publication number: WO 2022/259022

(56) References cited:
- EP-A1- 1 092 690
- EP-A1- 3 194 331
- WO-A1-2020/163513
- JP-A- H11 106 821
- US-A1- 2013 039 846
- US-A1- 2015 157 979
- KURODA M ET AL: "CO"2 reduction to methane and acetate using a bio-electro reactor with immobilized methanogens and homoacetogens on electrodes", ENERGY CONVERSION AND MANAGEMENT, ELSEVIER SCIENCE PUBLISHERS, OXFORD, GB, vol. 36, no. 6, 9 June 1995 (1995-06-09), pages 787 - 790, XP004039975, ISSN: 0196-8904, DOI: 10.1016/0196-8904(95)00122-T

## Description

### FIELD OF INVENTION

The present invention relates to carbon dioxide (CO₂) capture system and method and more specifically to a system and method for the carbon dioxide (CO₂) capture and utilization (CCU), converting the captured carbon dioxide (CO₂) to chemical compounds, like hydrogen (H₂) or methane (CH₄), by using metallic iron and/or metallic magnesium in anaerobic process.

In particular, the carbon dioxide (CO₂) capture and utilization method comprises a) alkaline solution with metallic iron and/or metallic magnesium to convert carbon dioxide (CO₂) to hydrogen (H₂) or b) alkaline solution with metallic iron and/or metallic magnesium and mix hydrogenotrophic methanogens to convert carbon dioxide (CO₂) to methane (CH₄).

### BACKGROUND

Carbon dioxide (CO₂), as the most significant anthropogenic greenhouse gas (GHG), substantially contributes to global warming and climate change. By the 2016 Paris Agreement has been firmly established that CO₂ is the primary driver of global warming and, for this reason, investor pressure and voluntary responses from many multinational firms and industries have been stimulated, creating significant opportunities for technological advances. In the European Union, the industrial plants are subject to the EU Emissions Trading System and have to reduce their GHG by 43% in 2030 compared to 2005. From 2021 onwards, if EU countries do not meet their decarbonization path up to 2030, they will have to purchase emission permits to make up for the shortfall in emission reduction which will be funded by the public budget.

To mitigate this, researchers and engineers have examined processes for transforming CO₂ into commodity chemicals and fuels; the process is also known as carbon dioxide (CO₂) capture and utilization (CCU). However, this is considered a challenge, as CO₂ presents kinetic and thermodynamic stability due to its centrosymmetric structure and the use of high-energy substances or processes is required for its reduction.

The most frequent carbon dioxide capture technologies are absorption, adsorption, membrane separation, and cryogenic technology. Among them, the most mature and most widely used technology is absorption, which captures CO₂ through chemical reactions.

Monoethanolamine (MEA) process has become the most widely used process for the CO₂ capture; however, the main disadvantages of MEA in CO₂ absorption are that MEA is volatile and easily degraded, leading to a large amount of solvent loss.

Another practical method to capture CO₂ is by absorbing it into alkaline aqueous solutions, like NaOH, and producing bicarbonate. The CO₂ absorption capacity of NaOH solution is higher than that of MEA, and NaOH is even more abundant and cheaper than MEA.

For example, the US patent application US 2010/0034724 A1 discloses a method of carbon dioxide capture, wherein in a step (a) anhydrous sodium carbonate is separated from a first aqueous solution formed by reacting carbon dioxide and an aqueous solution of sodium hydroxide. In step (b) the anhydrous Sodium carbonate is treated by causticization to generate carbon dioxide and sodium hydroxide. The first aqueous solution of step (a) is formed by scrubbing a gas containing carbon dioxide with an aqueous solution of Sodium hydroxide.

For example, the European patent application EP 3,673,972 A1 is related to a method and related device for capturing carbon dioxide gas by contacting it with a liquid alkaline solution, which react with the carbon dioxide and converts it into (bi)carbonate, and including a further processing step to convert the (bi)carbonate to an economically valuable chemical compound.

For example, the international application WO 2008/099252 A1 discloses an anaerobic process for converting CO₂ into methane. In one embodiment is described a module/phase comprising the following stages:
- CO₂ removal from flue gas in an extraction zone, by means of an alkaline solution of sodium minerals and CO₂ dissolution and hydration catalyzed by immobilized carbonic anhydrase;
- precipitation by sodium carbonate of the species resulting from CO₂ hydration and obtainment of an oversaturated sodium bicarbonate solution [...].

The problem of capturing carbon dioxide (CO₂) by absorbing it into alkaline aqueous solutions, like NaOH, producing bicarbonate is that bicarbonate has a high solubility, is stable and is hardly decarbonized. Moreover, NaOH, can hardly be regenerate from the bicarbonate.

Several studies have demonstrated the use of bicarbonate solution as a feed for microalgae, while other research have mixed the bicarbonate solution with calcium or magnesium waste to produce CaCO₃ and MgCO3, respectively.

See, for example, *"*A novel concept of bicarbonate-carbon utilization via an absorption-microalgae hybrid process assisted with nutrient recycling from soybean wastewater, Chunfeng Song, et al., Journal of Cleaner Production, Volume 237, 10 November 2019."

For example, the US patent application US 2007/0202032 A1, discloses a process for producing CaCO₃ or MgCO₃ from a feedstock comprising a Ca- or Mg- comprising mixed metal oxide, and to a process for the production of an aqueous solution of Ca(HCO₃)₂ or Mg(HCO₃)₂.

As may be understood from the cited examples, so far, a circular process that using alkaline aqueous solutions, like NaOH, can lead to the capture of CO₂, forming bicarbonate solution and producing Hydrogen, regenerating at the same time the alkaline NaOH solution, is not available from the art, and therefore is desirable.

For example, patent EP 3194331 B1 provides a synthesis method to produce hydrogen which is more economical and/or less energy consuming. According to the disclosed method, by using a hydrothermal process in the presence of carbonate ions, it is possible to produce hydrogen with high purity and yield. The capture of carbon dioxide is not envisaged in this case, but carbon dioxide performs a catalytic role in the reaction to let the aqueous composition comprising carbonate ions to react with the metallic iron under hydrothermal conditions.

The patent application WO2020163513A1 is an example of a system including a CO₂ capture device configured to receive a CO2-containing stream and including an aqueous alkaline solution comprising hydroxide and/or carbonate ions. The system also includes an electrolyzer, wherein a carbon-rich solution is received in an incoming stream to generate hydrogen, oxygen, and CO2 gas streams.

Furthermore, large industries, like power stations, cement plants and steel plants emit vast amounts of CO₂. More specifically, iron steel industries and cement industries, apart from the huge amount of CO₂, make use of siderite as a raw material in their process. For example, magnesium carbonate is used in the cement industry.

Hence a CCU process that produces as reaction by-product iron carbonate, like siderite, or magnesium carbonate, is indeed desirable because, they can be directly utilized as a raw material in steel industry.

Another potential field of application lies in the field of biogas upgrading. Biogas is the product of the degradation of organic matter in the absence of oxygen (anaerobically), and consists mainly of methane and carbon dioxide, and biogas upgrade is the process of separating methane from carbon dioxide and other gases from biogas. There are more than 17,736 biogas plants in Europe and therefore CO₂ capture and utilization from biogas increase its calorific value as well as its potential use.

There are many Carbon capture and utilization (CCU) and direct air capture (DAC) based companies that use mainly physicochemical processes. Therefore, being able to take advantage of a new low cost / efficient CCU process would be desirable for this type of business.

Finally, another potential application could be found in the generation of energy in remote places, even in spacecraft mission on Mars. For example, Mars mineral composition consists mainly of iron and magnesium minerals and the atmosphere is CO₂, and there is water-ice on its surface. Therefore, a method which exploits the abundance of carbon dioxide and of minerals on the soil of Mars could be a solution for an abundant energy generation directly on planet.

### SUMMARY

A specific object of the present invention is that of providing a system and method for capturing the carbon dioxide (CO₂) by converting it as soluble bicarbonate HCO₃- to H₂ using zero valent metal (Fe⁽⁰⁾ or Mg⁽⁰⁾) as defined in the appended set of claims.

In accordance with the present invention, there is thus provided a process by which carbon dioxide (CO₂) is absorbed in an alkaline solution, for example an aqueous solution comprising an alkali metal hydroxide, for example sodium hydroxide (NaOH), and soluble bicarbonate (NaHCO₃) is formed. Soluble bicarbonate (NaHCO₃) is first reacted with zero valent metal, consisting of commercial powder iron (Fe) or magnesium (Mg) ribbon and converted to hydrogen gas (H₂), comprising a concentration >97% H₂.

According to an aspect of the present invention, the capturing of carbon dioxide (CO₂) is used to generate hydrogen gas (H₂) for being directly used for energy purposes. According to the present invention, the reaction shows higher reaction rate for production of H₂ with higher bicarbonate concentration, for example 10-100 [gr NaHCO₃/L] compared to the same reaction with oxygen or with a lower bicarbonate concentration.

According to the present method, a circular process is created, and the solution after having reacted is reusable for CO₂ capture. In fact, the siderite, which is created on the outer surface of metal iron Fe⁽⁰⁾ after a first reaction, can be separated by the alkaline solution. By exposing it to citric acid (0.1M-0.5M) or other weak acids in a second reaction, the siderite in the outer surface of Fe⁽⁰⁾ is going to be removed. After this the citric acid solution is removed, and the remaining Fe⁽⁰⁾ is recyclable in the reaction for the H₂ production.

According to another embodiment, the siderite, instead of being removed from the outer surface of Fe⁽⁰⁾, it can be used as is, for example, as raw material for the steel industry or cement industry or as an iron scrap. With the same purpose, Magnesium carbonate can also be used as a raw material in the cement industry.

According to a further embodiment, the siderite can be used for other applications such as Phosphate absorption or heavy metals adsorption from wastewater.

According to another aspect not falling within the subject matter for which protection is sought, hydrogen gas (H₂), instead of being used for energy purposes, it can be used in another reaction for the production of methane (CH₄), which has more potential final use. For example, is known the use of H₂ from electrolysis to feed the hydrogenotrophic methanogens. The hydrogenotrophic methanogens convert H₂ and soluble CO₂ to methane (CH₄). According to an embodiment of the present method, not falling within the subject matter for which protection is sought, the hydrogen (H₂) generated in a first reaction is then directed in another reactor comprising bicarbonate solution and mix hydrogenotrophic methanogens. Experimental results have shown that in 4 days higher than 98% of CH₄ is obtainable by a system comprising CO₂ and Fe⁽⁰⁾ bicarbonate solution and mix hydrogenotrophic methanogens

According to still another embodiment not falling within the subject matter for which protection is sought, H₂ generated by the first reaction can be directed to a bioreactor comprising 'homoacetogenic' bacteria that converts H₂ and CO₂ to carboxylic acids, mainly acetic acid.

These and other objectives are fully achieved by virtue of the system and method for capturing carbon dioxide (CO₂) according to the present invention having the characteristics defined in independent claims 5 and 1.

Preferred embodiments of the invention are specified in the dependent claims, whose subject matter is to be understood as forming an integral or integrating part of the present description.

### BRIEF DESCRIPTION OF THE DRAWINGS

For a better understanding of the present invention, preferred embodiments, which are intended purely by way of example and are not to be construed as limiting, will now be described with reference to the attached drawings, where:
- Figure 1 is a diagram representing a first embodiment of the method of capturing carbon dioxide according to an aspect of the present invention.
- Figure 2 is a diagram representing a second embodiment of the method of capturing carbon dioxide according to another aspect of the present invention.
- Figure 3 is a diagram representing a third embodiment of the method of capturing carbon dioxide not falling within the subject matter for which protection is sought.
- Figure 4. Production of H₂ (mmol)/Fe(kg) from Fe⁽⁰⁾ (50gr/L) in solution (a) flushed with N₂, (b) flushed with CO₂ and (c) with 10gr NaHCO₃/L flushed with CO₂.
- Figure 5a. Production of H₂ (mmol)/Fe(kg) from Fe⁽⁰⁾ (25g/L) in solution (a) flushed with N₂, (b) flushed with CO₂, (c) with 25gr NaHCO₃/L flushed with CO₂ and (d) with 50gr NaHCO₃/L flushed with CO₂. Initial pH = 7.3.
- Figure 5b. Production of H₂ (%) from Fe⁽⁰⁾ (25g/L) in solution (a) flushed with N₂, (b) flushed with CO₂, (c) with 25gr NaHCO₃/L flushed with CO₂ and (d) with 50gr NaHCO₃/L flushed with CO₂. Initial pH = 7.3.
- Figure 6. Production of H₂ (%) in solution with 10gr NaHCO₃/L, flushed with CO₂ and with Fe⁽⁰⁾ concentration of 5 gr ^{/}L, 10 gr/L, 25 gr/L, 50gr/L.
- Figure 7. Production of H₂ (%) from Mg⁽⁰⁾ (2gr/L) in solution (a) exposed to air, (b) flushed with CO₂, (c) with 10gr NaHCO₃/L flushed with CO₂. Initial pH = 6.
- Figure 8. Production of H₂ (%) over time from Mg⁽⁰⁾ (2gr/L) in solution (a) flushed with CO₂, (b) with 10gr NaHCO₃/L flushed with CO₂, (c) with 20gr NaHCO₃/L flushed with CO₂ and (d) 40gr NaHCO₃/L flushed with CO₂. Initial pH = 7.3.
- Figure 9. Production of H₂ (%) over time from Fe⁽⁰⁾ (50 gr/L) in solution of NaHCO₃ previously formed from 0.35M NaOH and CO₂. Initial pH = 7.3.
- Figure 10. Gibbs free energy change ΔG (KJ/mol) for metallic iron reaction with H₂O and N₂ at headspace and Gibbs free energy change ΔG (KJ/mol) for metallic iron reaction with H₂O, bicarbonate and CO₂ at headspace.

### DETAILED DESCRIPTION

The following discussion is presented to enable a person skilled in the art to make and use the invention. Various modifications to the embodiments will be readily apparent to those skilled in the art, without departing from the scope of the present invention as claimed. Thus, the present invention is not intended to be limited to the embodiments described therein, but it has to be accorded the widest scope consistent with the principles and features disclosed herein and defined in the appended claims.

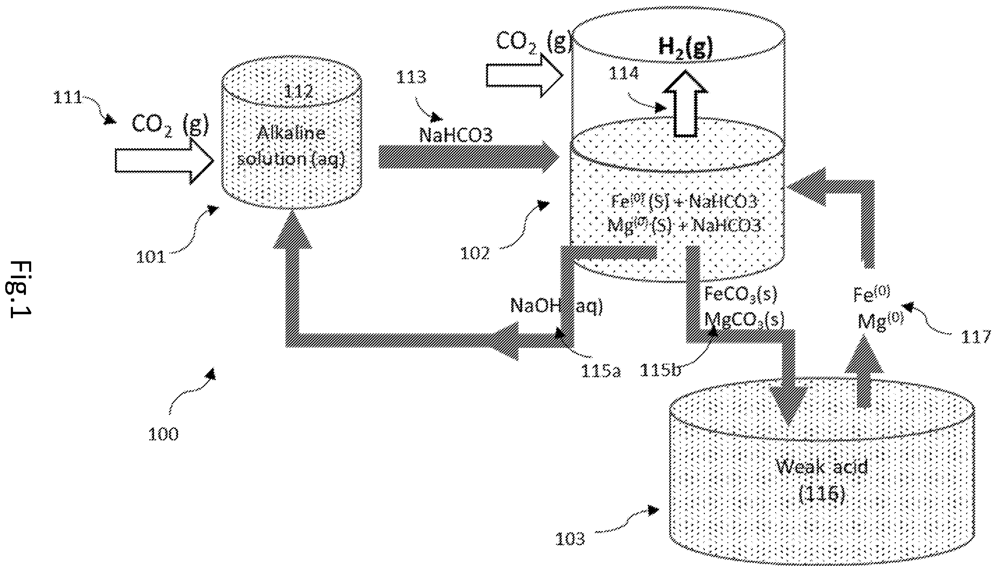

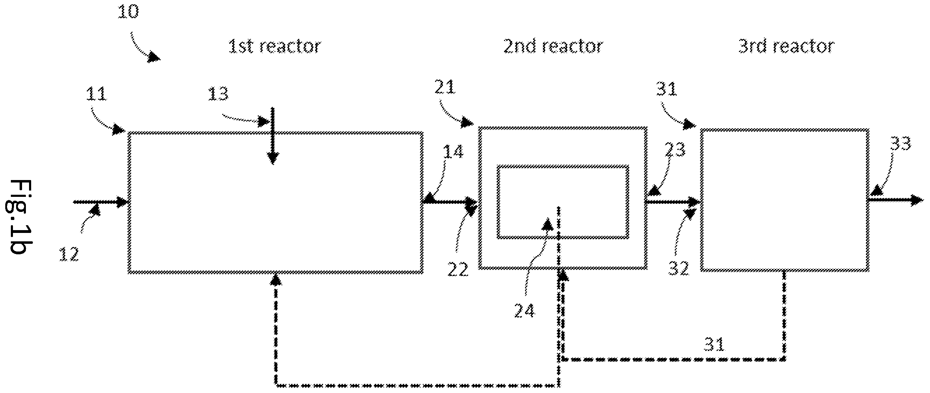

According to Fig.1, a process (100) of capturing and converting carbon dioxide (CO₂) is described comprising a first step (101) by which carbon dioxide, CO₂ (111) is absorbed in an alkaline solution (112), typically an aqueous solution comprising, for example, sodium hydroxide (NaOH), with a concentration comprised between 0,1M to 1,3M, preferably with a concentration of 0.9M, having an initial pH > 10, and as a result soluble bicarbonate, NaHCO₃ (113) , having pH between 7-8, is formed, according for example to the following reaction formula (1):

1) NaOH (aq) + CO₂ → NaHCO₃ (aq)

Bicarbonate (NaHCO₃), as reaction product, is stable and is hardly decarbonized. To overcome this problem, a second step (102) is used, wherein bicarbonate (113) is reacted with zero valent metal, for example commercial powder Fe, to produce hydrogen gas, H₂ (114), according for example to the reaction formula (2):

2) Fe⁽⁰⁾ (s) + HCO₃- (aq) + H₂O → FeCO₃(s) + H₂(g) + OH⁻ (aq).

As shown in Fig.1, in step (102) the bicarbonate solution reacts with metallic Fe⁽⁰⁾ and H₂ (114) is generated in the headspace. Also, the initial CO₂ in the headspace is moved to the liquid solution and reacts with metallic Fe⁽⁰⁾ therefore the headspace contains final >98% H₂. As shown by the reaction formula (2), the second reaction step (102) comprises the production of solid metal carbonate, for example FeCO₃, as by-product increasing alkalinity (OH⁻).

Instead of commercial powder Fe, a scrap-Fe can be used, which will significantly reduce the process cost; however, the reaction rate is 4-5 times slower but the final H₂ concentration is still more than 98%. Furthermore, Magnesium (Mg⁽⁰⁾) ribbon can be used instead of Fe⁽⁰⁾ at a lower concentration than Fe, according to the reaction formula (3):

3) Mg⁽⁰⁾ (s) + HCO₃- (aq) + H₂O → MgCO₃(s) + H₂(g) + OH- (aq) .

Both the reactions (2) and (3) occur in anaerobic carbonate ambient conditions using metallic Fe (powder) or scrap Fe or Magnesium (Mg) ribbon.

According to an aspect of the present invention, the reaction become faster (higher reaction rate) in the production of H₂ in the presence of high concentration of bicarbonate, for example (10-100gr NaHCO₃/L). Final gas composition comprises a Hydrogen (H₂) concentration > 98% after a reaction time ranging from 1 to 96 hours.

The rate and the yield of reaction depend on several conditions, comprising:
- Fe or Mg concentration,
- surface area of Fe or Mg,
- bicarbonate concentration,
- temperature,
- initial pH,
- initial headspace gas,
- agitation,
- initial pressure,
- headspace volume.

According to an embodiment of the present method, the reaction is more thermodynamically favorable (compared to the same conditions with oxygen or with low bicarbonate) and increases the reaction rate. Specifically using Fe⁽⁰⁾ powder, the reaction rate (H₂mmol/Fe(kg)·(h)) of H₂ is 1.5 - 2.5 higher at higher bicarbonate concentration compared to the condition without bicarbonate or to the use of only CO₂. On the other hand, the reaction rate (H₂mmol/Fe(kg)·(h)) of high bicarbonate is 250 - 350 higher compared with the Fe⁽⁰⁾ powder exposed to water and nitrogen (N₂). For example, the reaction (2) is very slow when N₂ is used instead of CO₂. However, bicarbonate solution and CO₂ in the gas phase (at initial conditions) result in a significantly higher H₂ production rate than using water and CO₂ in the gas phase. In this process according to reaction (2), no external energy is needed for the reaction to occur.

At the end of the reactions (2) or (3), pH is alkaline (pH 8.5 - 9.5), therefore once the alkaline solution is separated from Fe or Mg, it can be recycled to be used again for more CO₂ absorption as in reaction (1) and then the final pH will be drop to 7.0 - 8.

According to a first aspect of the present invention, the hydrogen gas, H₂ (114) generated according to the reaction formula (2) can be directly utilized for energy purpose.

According to an embodiment of the present method the metal carbonate (115b) obtained in the reaction step (102) is separated obtaining an aqueous alkaline solution, comprising for example NaOH (115a), wherein said aqueous alkaline solution is recycled in the reaction step (101). According to an embodiment of the present method, a solid metal carbonate (115b), for example the siderite (FeCO₃), is created in the outer surface of Fe⁰.

The solid metal carbonate (115b) can be removed, by a further a step (103), by which weak acid (116), comprising citric acid or oxalic acid, is used with concentration in the range 0.1M - 0.5M, so that the remaining zero valent metal (117) can be recycled in the reaction step (102) and the reaction according to the formula (2) can be initiated again. The generation of carbonate (115b) on the surface of Fe or Mg is indicated by the reduction of the concentration of H₂ from the gas phase to lower than 95% (after several batch cycles). According to a preferred embodiment, after removal of carbonate the remaining solid (FeCO₃ or MgCO₃) is exposed to citric acid or weak acid for several hours so the external FeCO₃ is removed and the inner Fe can react again in step (102).

According to another embodiment of the present method, the solid FeCO₃ (s) or MgCO₃ (s) instead of being directed to step (103) to be removed from the outer surface, it is used as a raw material in steel industry or cement industry or they are commercialized as iron scrap.

According to a further embodiment of the present method, the siderite is used in other applications, such as Phosphate absorption or heavy metals adsorption from wastewater.

According to a second aspect not falling within the subject matter for which protection is sought, the hydrogen gas (H₂) generated according to the reaction formula (2) or (3), instead to be used for energy purposes, is directed to a bioreactor comprising Hydrogenotrophic methanogens for methane (CH₄) production or Homoacetogenic bacteria for acetic acid (CH₃COOH) production. The bioreactor should also contain sodium bicarbonate (NaHCO₃) solution from reaction (1).

The microbial inoculum can be a mixed culture such as anaerobic granular sludge or anaerobic sludge from the digester. The inoculum can be pre-exposed to favorable conditions to enrich the hydrogenotrophic methanogens or homoacetogens, depending on the final product.

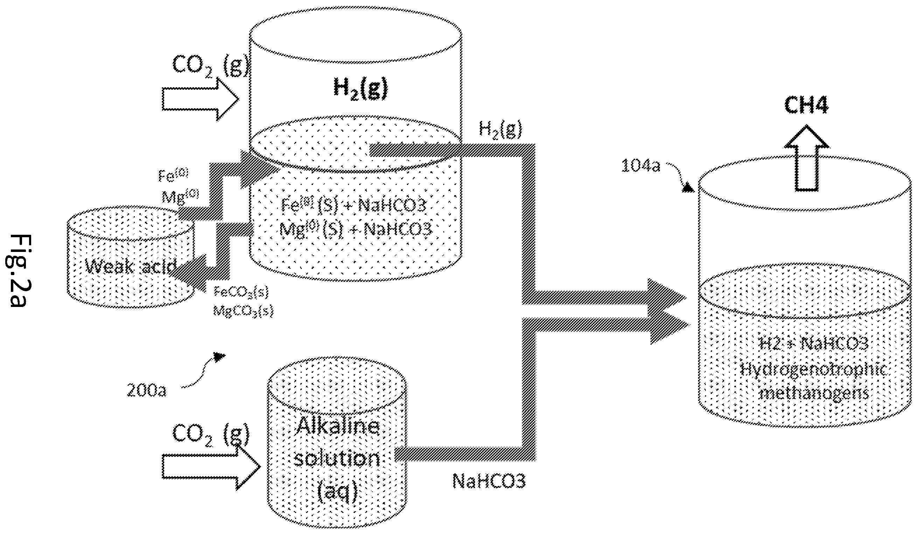

According to an embodiment of the present method (200a) not falling within the subject matter for which protection is sought, the hydrogen gas (H₂) generated according to the reaction formula (2) or (3), is directed to a bioreactor (104a) comprising hydrogenotrophic methanogens, as shown in Fig.2a, wherein the reagents H₂ and soluble CO₂ are converted to CH₄, according to a reaction formula (4):

4) 3H₂ +HCO₃⁻ → CH₄ + 3H₂O

Methane (CH₄) has more potential final uses than H₂, said uses comprising energy, fuel vehicle, storage in natural gas grid.

Conventionally, in the process of using hydrogenotrophic methanogens to convert CO₂ to CH₄, the external H₂ gas is added from a separate electrochemical process unit (electrolysis), which splits water into H₂ gas and O₂ by utilizing surplus renewable electric power. One of the current drawbacks to use this process is the relatively high cost of electrolysis and the integration of various systems such as electrolysis process and the H₂ addition to anaerobic digester/bioreactor. In other studies, microbial electrosynthesis has been used for biogas upgrading; however, the main challenges in this case are the cost reduction and the absence of pilot-scale and full-scale demonstration for the process.

According to an aspect not falling within the subject matter for which protection is sought, in the process of using hydrogenotrophic methanogens to convert CO₂ to CH₄, the use of H₂ as generated from the reaction (2) and/or (3) is proposed. By experimental results, it has been shown that in 4 days higher than 98% of CH₄ can be obtained starting by a system comprising CO₂ and Fe^{(o)}.

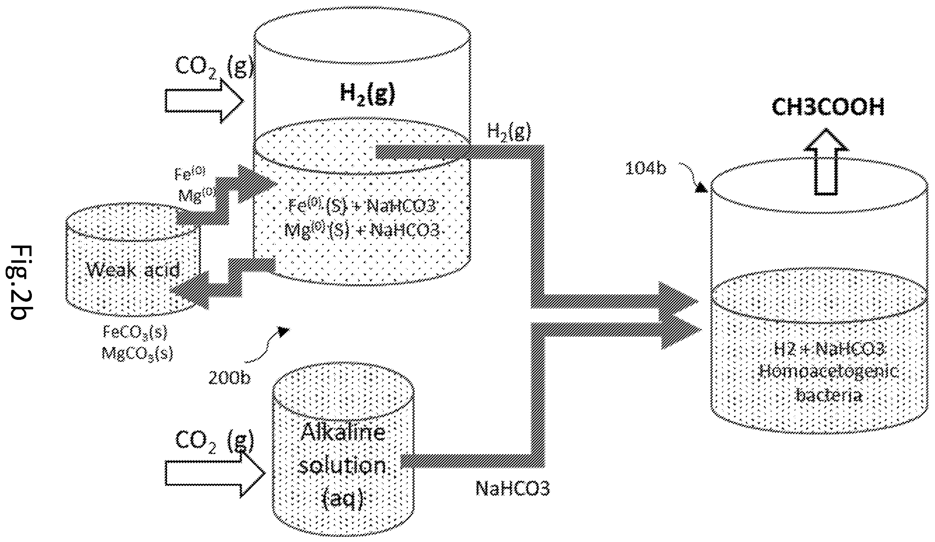

Alternatively, according to another embodiment of the present method (200b) not falling within the subject matter for which protection is sought, the gaseous hydrogen (H₂) can be directed to a bioreactor (104b, Fig.2b) comprising homoacetogenic bacteria to convert H₂ and CO₂ to carboxylic acids, for example acetic acid (CH₃COOH) according to a reaction formula (5):

5) 2HCO₃- + 4.5H₂ → CH₃COO- + 4H₂O

At the end of reaction (4) or reaction (5) pH is alkaline and the solution can be recycled for CO₂ absorption (as previously descripted).

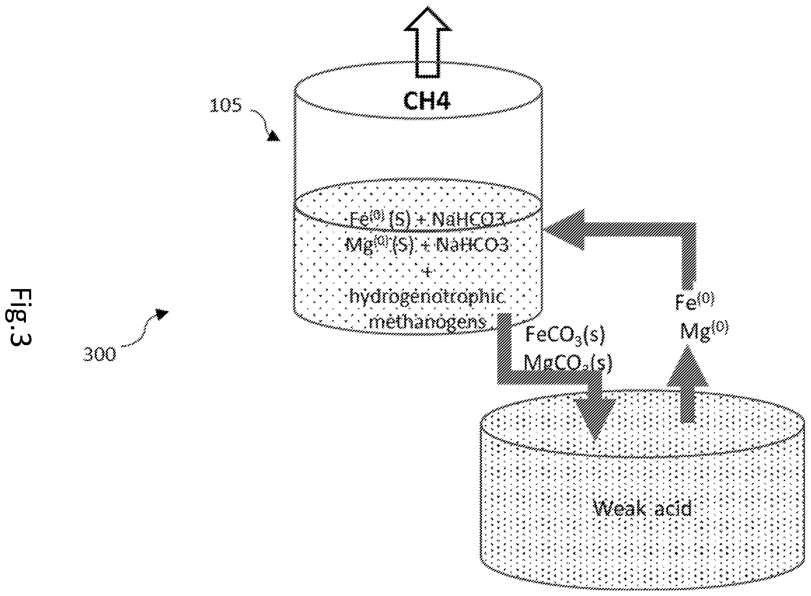

According to another embodiment (300) of the present method not falling within the subject matter for which protection is sought, the reactions (2) or (3) can take place "in-situ" with the reaction (4) in one and the same reactor (105, Fig.3), reacting them with hydrogenotrophic methanogens in the same reactor, in such a way to produce a final gas with CH₄ > 97%, according to the reaction formula (6) or (7):

6) 4Fe⁽⁰⁾ + 4HCO₃⁻ + 4H⁺ + CO₂ → 4FeCO₃ + CH₄ + 2H₂O;

7) Mg⁽⁰⁾ + 4HCO₃⁻ + 4H⁺ + CO₂ → 4MgCO₃ + CH₄ + 2H₂O.

The limitation of using this "in-situ" process is the inhibition of sodium to the microorganisms; therefore, the sodium bicarbonate should be less than 30gr NaHCO3/L. For higher concentrations of sodium bicarbonate, halophilic microorganisms has to be used.

The FeCO₃ formation can be removed with citric acid as already shown in the foregoing. The reaction (6) or (7) will require about 3-6 days to be completed (CH4 > 97%).

Referring to Fig.1b, an apparatus (10) for carrying out the method of capturing and converting carbon dioxide (CO₂) as described in the foregoing comprises at least a first reactor (11) and a second reactor (21). The first reactor (11) comprises a first inlet module (12) for the gaseous carbon dioxide (CO₂), a second inlet module (13) for an aqueous alkaline solution comprising an alkali metal hydroxide, at least one outlet module (14), and is configured to absorb the gaseous carbon dioxide in the aqueous alkaline solution comprising an alkali metal hydroxide such that the carbon dioxide reacts with the alkali-metal hydroxide to form a bicarbonate. The second reactor (21) comprises an inlet module (22) for the bicarbonate solution, at least one outlet module (23), and is operatively connected to said first reactor (11) and is configured to bring in contact the bicarbonate solution with a zero valent metal species to form a reaction mixture of H₂, solid metal carbonate and aqueous alkaline solution.

According to a preferred embodiment of said apparatus (10), said second reactor (21) comprises a liquid/solid separation module (24) configured to separate the reaction mixture into a regenerated aqueous alkaline solution and a solid metal carbonate, wherein said regenerated aqueous alkaline solution is recycled in the first reactor (11).

According to an embodiment, said apparatus comprises a third reactor (31), having an inlet module (32) and at least one outlet module (33), and is configured to receive Fe carbonate from the second reactor (21), bring in contact said Fe carbonate with either citric acid or oxalic acid to form zero valent Fe, wherein said zero valent Fe is recycled in the second reactor (21).

### Experimental results

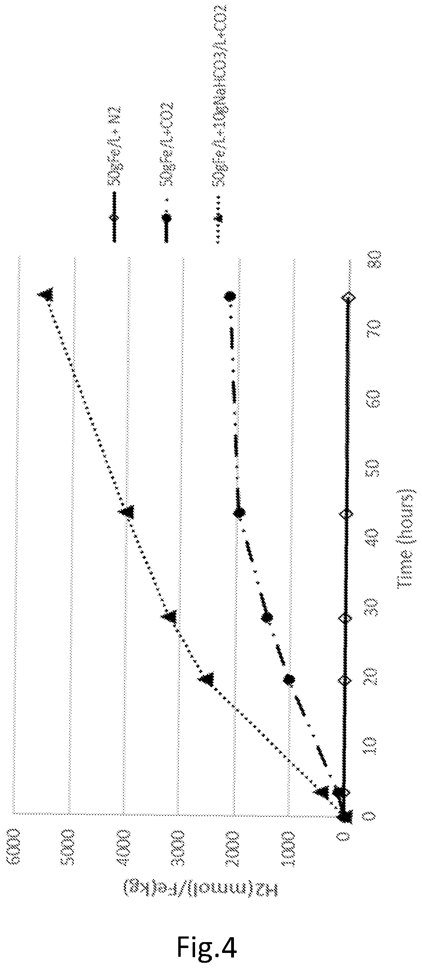

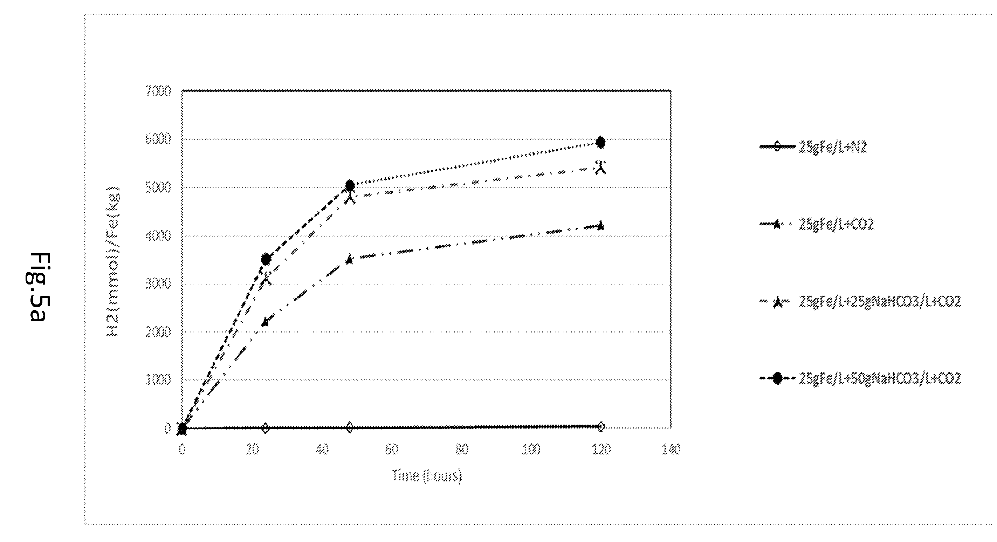

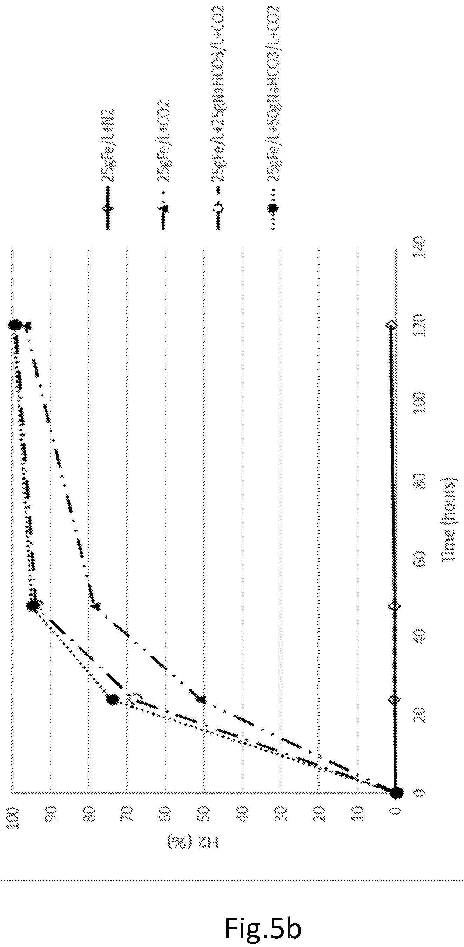

1) Examined parameters: N₂, CO₂ and 10gr NaHCO₃/L. with CO₂ at 50gr Fe⁽⁰⁾/L. Experimental condition: Fe⁽⁰⁾ (50g/L), serum bottles (165 ml), working volume 65ml of water, headspace 100 ml, temperature 33°C, initial pH 6, agitation 100 rpm.
As can be seen from the chart in Fig. 4, representing the H₂ mol generation Vs time, when the system was flushed with N₂ (for 2 min), it resulted in negligible H₂ production after 75 hours. On the contrary, when the system was a) flushed with CO₂ or b) with CO₂ plus 10gr NaHCO₃/L in the solution, it resulted in a dramatically higher H₂ (mmol) generated over time. The presence of 10gr NaHCO₃/L in addition to being flushed with CO₂ resulted in double H₂ mmol/Fe(kg) than in the samples flushed with CO₂ without extra 10gr NaHCO3/L.
2) Examined parameters: N₂, CO₂, 25gr NaHCO₃/L with CO₂ and 50gr NaHCO₃/L with CO₂ at 25 gr Fe⁽⁰⁾/L.
Experimental condition: Fe⁽⁰⁾ (25gr/L), serum bottles (165 ml), working volume 50 ml of water, headspace 115 ml, temperature 33°C, initial pH 7.3, agitation 100 rpm.
3) The chart in Fig.5a shows that the higher the bicarbonate concentration, the higher the H₂(mmol)/Fe(kg) generated over time. Again, the use of N₂ instead of CO₂ results in negligible H₂ production Fig 5a and Fig.5b. The samples with bicarbonate solution (25 and 50gr NaHCO₃/L) produced higher than 94% H₂ in 48 hours, whereas the samples flushed with CO₂ without the addition of NaHCO₃ generated 78% H₂ . Examined parameters: N₂, CO₂, 25gr NaHCO₃/L with CO₂ and 50gr NaHCO₃/L with CO₂, 75gr NaHCO₃/L with CO₂, 100gr NaHCO₃/L with CO₂ at 25gFe⁽⁰⁾/L. Experimental conditions: Fe⁽⁰⁾ (25g/L), serum bottles (165 ml), working volume 50 ml of water, headspace 115 ml, temperature 33°C, initial pH 7.5-7.7, agitation 100 rpm.

**Table 1 shows the production of H₂ (mmol)/Fe(kg).h from Fe⁽⁰⁾ (25gr/L) under various NaHCO₃ concentrations with a) flushed with N₂ without NaHCO₃ b) flushed with CO₂ without NaHCO₃ c) 25gr NaHCO₃/L and flushed with CO₂ d) 50gr NaHCO₃/L and flushed with CO₂.**

| | Rate of the reaction after 24 h H2 (mmol)/Fe(kg).h |
|---|---|
| Fe(0) exposed to N₂ | 0.22 |
| Fe(0) exposed to CO₂ | 101.7 |
| Fe(0) exposed to 25g NaHCO₃/L + CO₂ | 151.5 |
| Fe(0) exposed to 50g NaHCO₃/L + CO₂ | 160.8 |
| Fe(0) exposed to 75g NaHCO₃/L + CO₂ | 173.1 |
| Fe(0) exposed to 100g NaHCO₃/L + CO₂ | 198.8 |

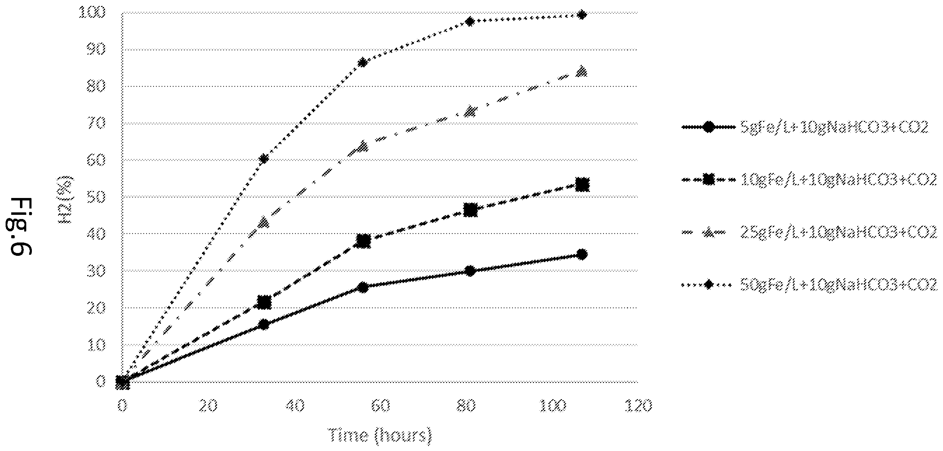

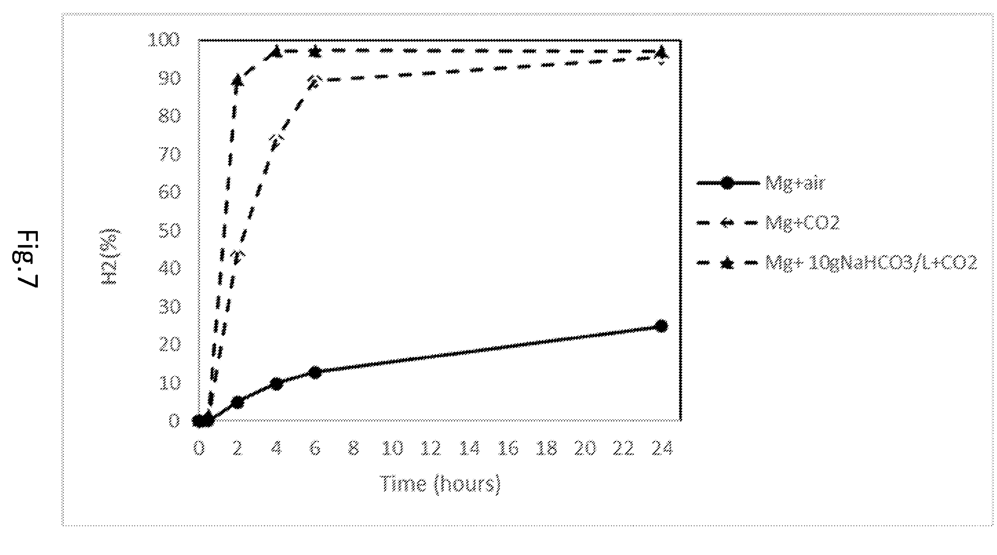

The rate H₂ (mmol)/Fe(kg).h of the reaction for Eq 2a for Fe⁽⁰⁾ was measured after 24 h. The higher the bicarbonate concentration, the higher the rate for higher production, as shown in Table 1. In the samples flushed with CO₂, the presence of 100 gr NaHCO₃/L almost double the reaction rate compared with no extra NaHCO₃ added in the solution.
4) Examined parameters: Concentrations of Fe⁽⁰⁾ (5, 10, 25, 50 gr/L ) under 10gr NaHCO₃ and CO₂.
Experimental conditions: Fe⁽⁰⁾ (50g/L), serum bottles (165 ml), working volume 65 ml of water, headspace 100 ml, temperature 33°C, initial pH 6, agitation 100 rpm. Results from section Fig.6 show that the higher concentrations of powder Fe resulted in higher production of H₂. At 50gr Fe/L with 10gr NaHCO₃/L, the H₂ is higher than 97% after 81 hours, whereas at this time point for 25gr Fe/L, the H₂ concentration was 84%. However, as shown in Fig 5b, the samples with 25gr Fe/L and 25gr NaHCO₃/L generated higher than 94% H₂ in 48 h. With 50g/L Fe⁽⁰⁾ and 75gr/L NaHCO₃ higher than 92% and 99% H₂ in 24 and 48 hours, are respectively produced. Therefore, both the Fe⁽⁰⁾ and NaHCO₃ positively contribute to H₂ production in the reaction, although NaHCO₃ concentrations had a more profound positive effect than Fe.
5) Examined parameters: exposure to Magnesium ribbon in air, in CO₂ ambient and CO₂ + NaHCO₃ ambient.
Experimental conditions: Mg⁽⁰⁾ ribbon (2gr/L), serum bottles (125 ml), working volume 70 ml of water, headspace 55 ml, temperature 33°C, initial pH 6, agitation 100 rpm.
As shown in Fig.7, after 2 hours, the presence of bicarbonate and CO₂ resulted in a higher rate of H₂ production (around 90% H₂), whereas the exposure of the samples to CO₂ resulted in 43% H₂ in 24 hours.

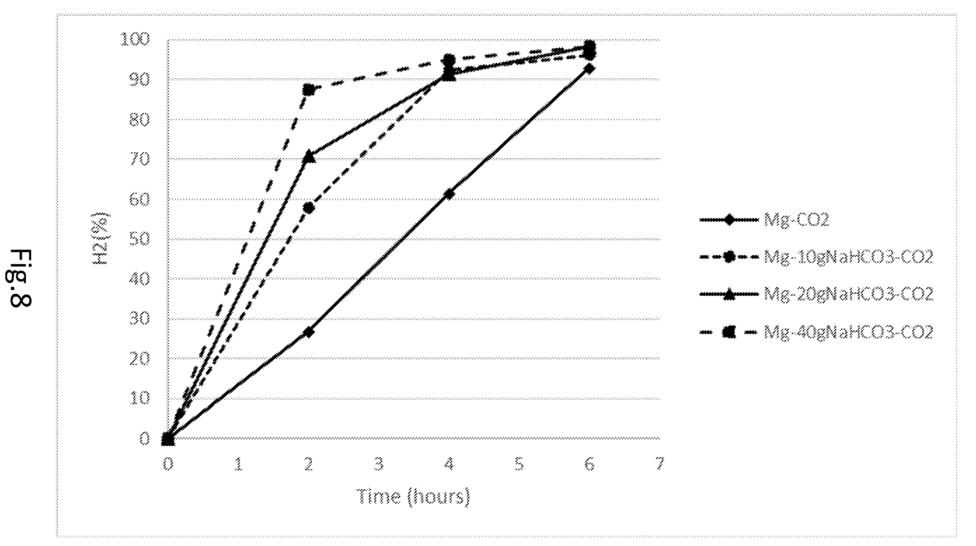

6) Examined parameters: exposure to Magnesium ribbon to CO₂, CO₂ + 10g NaHCO₃/L, CO₂ + 20g NaHCO₃/L and CO₂ + 40gNaHCO3/L.
Experimental conditions: Mg⁽⁰⁾ ribbon (2gr/L), serum bottles (125 ml), working volume 70 ml of water, headspace 55 ml, temperature 33°C, initial pH 7.2, agitation 100 rpm.
As shown in Fig 8, the higher the amount of bicarbonate, the higher the H₂ production over time. After 24 hours a crystal was formed on the metal surface. The crystal was identified to be a carbonate called Nesquehonite (MgCO₃.3H₂O).

7) Examined parameters: temperature (T) = 4°C, 20°C, 33°C.
Experimental conditions: same as experiment n.6 with 10gr NaHCO₃/L.
The reaction rate was:
   - 2856.4 H₂ mmol/Fe(Kg)·hours at T = 4°C,
   - 4314.4 H₂ mmol/Fe(Kg)·hours at T = 20°C,
   - 4746.9 H₂ mmol/Fe(Kg)·hours at T = 33°C.
At 4°C the reaction rate is decreased however a substantial amount of H₂ can be generated.

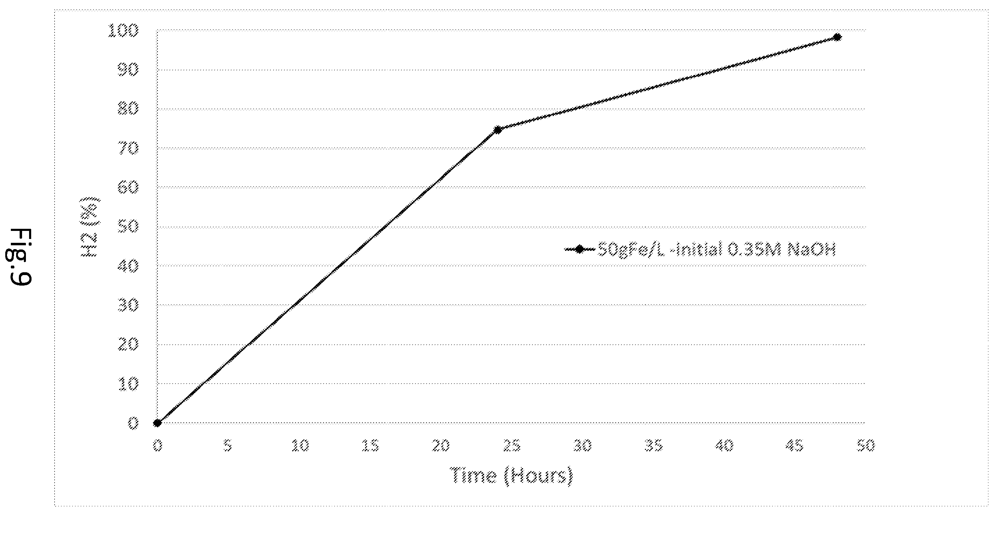

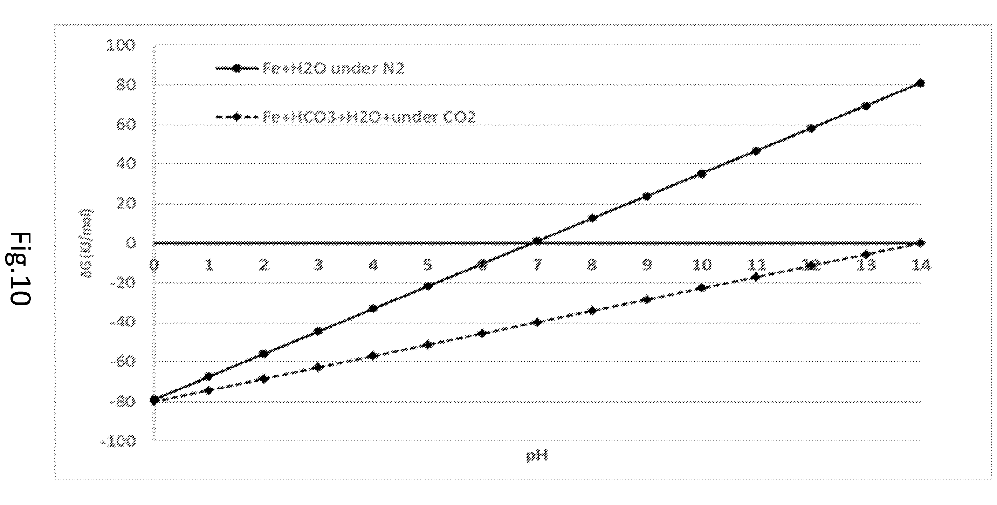

8) Examined parameters: Concentrations of Fe⁽⁰⁾ (50 gr/L), NaHCO₃ formed from initial 0.35 M NaOH continuously flushed with CO₂.
Experimental conditions: Fe⁽⁰⁾ (50g/L), serum bottles (125 ml), working volume 80 ml of water, headspace 45 ml, temperature 33°C, initial pH 7.3, agitation 100 rpm.
Results from section Fig.9 show that the H₂ is higher than 98% in 48 hours. At higher initial NaOH and higher initial Fe⁽⁰⁾ concentration the reaction occurs at a higher rate. Examined parameters: metallic iron reaction with H₂O and N₂ at headspace by varied pH 0-14 conditions (temperature 25⁰C, all concentrations 1 M and all partial pressures 1 atm) . Gibbs free energy change, ΔG (KJ/mol) is shown in Fig.10. The reaction (formula 8)

   8) Fe⁽⁰⁾ + 2H₂O → Fe²⁺ + H₂ + 2OH⁻

   becomes unfavorable at pH higher than 7.
In Fig 10, the Gibbs free energy change ΔG (KJ/mol) for metallic iron reaction (formula 2) with H₂O, bicarbonate and CO₂ at varied pH 0-14 (temperature 25⁰C, all concentrations 1 M and all partial pressures 1 atm) is also shown. In the presence of bicarbonate the reaction (formula 2) takes place even at alkaline pH.

Finally, it is clear that numerous modifications and variants can be made to the present invention, all falling within the scope of the invention, as defined in the appended claims.

## Claims

1. A process (100) for capturing and converting carbon dioxide (CO₂) comprising:
- a first reaction step (101) wherein a flux of gaseous carbon dioxide (CO₂) is absorbed in an aqueous alkaline solution comprising an alkali metal hydroxide, wherein the carbon dioxide (CO₂) reacts with the alkali metal hydroxide to form a bicarbonate, obtaining a bicarbonate solution,
- a second reaction step (102) wherein said bicarbonate solution is converted into hydrogen (H₂) **characterized in that**
in the second reaction step (102) said bicarbonate in the bicarbonate solution reacts with a zero valent metal species under anaerobic conditions to produce said hydrogen gas H₂ and an exhaust alkaline solution,
**and in that** said second reaction step (102) comprises the production of solid metal carbonate on the surface of zero valent metal as by-product, wherein said zero valent metal species is selected from the group comprising metallic Fe, scrap Fe, metallic Mg, Mg ribbon, and said solid metal carbonate is Fe carbonate or Mg carbonate,
said metal carbonate being separated (103) obtaining an aqueous alkaline solution, said aqueous alkaline solution being recycled in the reaction step (101) of absorbing the flux of gaseous carbon dioxide in the aqueous alkaline solution.

2. The process according to claim 1 **characterized in that** said aqueous alkaline solution is an alkaline solution containing NaOH with a concentration comprised between 0,1M to 1,3M, preferably with a concentration of 0.9M.

3. The process according to any one of the preceding claims **characterized in that** said bicarbonate solution has a pH between 7 and 8.

4. The process according to any of the preceding claims **characterized in that** said separated metal carbonate is treated with either citric acid or oxalic acid to obtain zero valent metal, wherein said zero valent metal is recycled in the second reaction step (102).

5. An apparatus (10) for capturing and converting carbon dioxide (CO₂) according to claims 1-4 comprising:
a first reactor (11) having a first inlet module (12) for gaseous carbon dioxide, a second inlet module (13) for an aqueous alkaline solution comprising an alkali metal hydroxide, at least one outlet module (14), configured to dissolve the gaseous carbon dioxide in the aqueous alkaline solution comprising an alkali metal hydroxide such that the carbon dioxide reacts with the alkali-metal hydroxide to form a bicarbonate,
**characterized in that**
the apparatus comprises a second reactor (21), having an inlet module (22) for the bicarbonate solution, at least one outlet module (23), wherein said second reactor (21) is operatively connected to said first reactor (11) and is configured to bring in contact the bicarbonate solution with a zero valent metal species under anaerobic condition to form a reaction mixture of H₂, solid metal carbonate and aqueous alkaline solution
**and in that** said second reactor (21) comprises a liquid/solid separation module (24) configured to separate the reaction mixture into a regenerated aqueous alkaline solution and a solid metal carbonate, wherein said regenerated aqueous alkaline solution is recycled in the first reactor (11).

6. The apparatus according to claim 5 comprising a third reactor (31), having an inlet module (32) and at least one outlet module (33), said third reactor (31) being configured to receive Fe carbonate from the second reactor (21), bring in contact said Fe carbonate with either citric acid or oxalic acid to form zero valent Fe, wherein said zero valent Fe is recycled in the second reactor (21).

## Patentansprüche

1. Verfahren (100) zum Auffangen und Umwandeln von Kohlendioxid (CO₂), umfassend:
- einen ersten Reaktionsschritt (101), in dem ein Strom von gasförmigen Kohlendioxid (CO₂) in eine wässrige alkalische Lösung absorbiert wird, die ein Alkalimetallhydroxid enthält, wobei das Kohlendioxid (CO₂) mit dem Alkalimetallhydroxid unter Bildung eines Bicarbonats reagiert, wodurch eine Bicarbonatlösung entsteht,
- einen zweiten Reaktionsschritt (102), in dem die Bicarbonatlösung in Wasserstoff (H₂) umgewandelt wird,
**dadurch gekennzeichnet, dass**
im zweiten Reaktionsschritt (102) das Bicarbonat in der Bicarbonatlösung unter aeroben Bedingungen mit einer nullwertigen Metallspezies reagiert, um das Wasserstoffgas H₂ und eine erschöpfte alkalische Lösung zu erzeugen,
**und dass** der zweite Reaktionsschritt (102) die Erzeugung von festem Metallcarbonat auf der Oberfläche eines nullwertigen Metalls als Nebenprodukt umfasst, wobei die nullwertige Metallspezies aus der Gruppe ausgewählt ist, die aus metallischem Fe, Fe-Schlacke, metallischem Mg und Mg-Band besteht, und dass das feste Metallcarbonat Fe-Carbonat oder Mg-Carbonat ist,
wobei das Metallcarbonat abgetrennt (103) wird, um eine wässrige alkalische Lösung zu erhalten, wobei die wässrige alkalische Lösung durch Absorption des Kohlendioxidgasstroms in der wässrigen alkalischen Lösung in den Reaktionsschritt (101) zurückgeführt wird.

2. Verfahren nach Anspruch 1, **dadurch gekennzeichnet, dass** die wässrige alkalische Lösung eine alkalische Lösung ist, die NaOH mit einer Konzentration zwischen 0,1 M und 1,3 M, vorzugsweise mit einer Konzentration von 0,9 M, enthält.

3. Verfahren nach einem der vorhergehenden Ansprüche, **dadurch gekennzeichnet, dass** die Bicarbonatlösung einen pH-Wert zwischen 7 und 8 aufweist.

4. Verfahren nach einem der vorhergehenden Ansprüche, **dadurch gekennzeichnet, dass** das abgetrennte Metallcarbonat mit Zitronensäure oder Oxalsäure behandelt wird, um ein nullwertiges Metall zu erhalten, wobei das nullwertige Metall in den zweiten Reaktionsschritt (102) zurückgeführt wird.

5. Vorrichtung (10) zum Auffangen und Umwandeln von Kohlendioxid (CO₂) nach Ansprüchen 1 bis 4, umfassend:
einen ersten Reaktor (11) mit einem ersten Einlassmodul (12) für Kohlendioxidgas, einem zweiten Einlassmodul (13) für eine wässrige alkalische Lösung, die ein Alkalimetallhydroxid enthält, und mindestens einem Auslassmodul (14), das so konfiguriert ist, dass es das Kohlendioxidgas in der wässrigen alkalischen Lösung, die ein Alkalimetallhydroxid enthält, löst, sodass das Kohlendioxid mit dem Alkalimetallhydroxid reagiert und ein Bicarbonat bildet,
**dadurch gekennzeichnet, dass**
die Vorrichtung umfasst einen zweiten Reaktor (21) mit einem Einlassmodul (22) für die Bicarbonatlösung und mindestens einem Auslassmodul (23), wobei der zweite Reaktor (21) operativ mit dem ersten Reaktor (11) verbunden ist und so konfiguriert ist, dass die Bicarbonatlösung unter anaeroben Bedingungen mit einer nullwertigen Metallspezies in Kontakt gebracht wird, um ein Reaktionsgemisch aus H₂, festem Metallcarbonat und einer wässrigen alkalischen Lösung zu bilden
**und dass** der zweite Reaktor (21) ein Flüssig/Feststoff-Trennmodul (24) umfasst, das so konfiguriert ist, dass es das Reaktionsgemisch in eine regenerierte wässrige alkalische Lösung und ein festes Metallcarbonat trennt, wobei die regenerierte wässrige alkalische Lösung in den ersten Reaktor (11) zurückgeführt wird.

6. Vorrichtung nach Anspruch 5, umfassend einen dritten Reaktor (31) mit einem Einlassmodul (32) und mindestens einem Auslassmodul (33), wobei der dritte Reaktor (31) so konfiguriert ist, dass er Fe-Carbonat aus dem zweiten Reaktor (21) aufnimmt, das Fe-Carbonat mit Zitronensäure oder Oxalsäure in Kontakt bringt, um nullwertiges Fe zu bilden, wobei das nullwertige Fe in den zweiten Reaktor (21) zurückgeführt wird.

## Revendications

1. Procédé (100) de capture et de conversion de dioxyde de carbone (CO₂) comprenant :
- une première étape de réaction (101) dans laquelle un flux de dioxyde de carbone gazeux (CO₂) est absorbé dans une solution alcaline aqueuse comprenant un hydroxyde de métal alcalin, le dioxyde de carbone (CO₂) réagissant avec l'hydroxyde de métal alcalin pour former un bicarbonate, ce qui donne une solution de bicarbonate,
- une deuxième étape de réaction (102) dans laquelle ladite solution de bicarbonate est convertie en hydrogène (H₂),
**caractérisé en ce que**
dans la deuxième étape de réaction (102), ledit bicarbonate dans la solution de bicarbonate réagit avec une espèce métallique de valence zéro dans des conditions anaérobies pour produire ledit hydrogène gazeux H₂ et une solution alcaline évacuée,
**et que** ladite deuxième étape de réaction (102) comprend la production de carbonate métallique solide à la surface d'un métal de valence zéro comme sous-produit, ladite espèce métallique de valence zéro étant choisie dans le groupe comprenant Fe métallique, Fe de scories, Mg métallique, Mg en ruban, et ledit carbonate métallique solide est un carbonate de Fe ou un carbonate de Mg, ledit carbonate métallique est séparé (103) pour obtenir une solution alcaline aqueuse, ladite solution alcaline aqueuse étant recyclée dans l'opération de réaction (101) en absorbant le flux de dioxyde de carbone gazeux dans la solution alcaline aqueuse.

2. Procédé selon la revendication 1, **caractérisé en ce que** ladite solution alcaline aqueuse est une solution alcaline contenant NaOH avec une concentration comprise entre 0,1M et 1,3M, de préférence avec une concentration de 0,9M.

3. Procédé selon l'une quelconque des revendications précédentes, **caractérisé en ce que** ladite solution de bicarbonate a un pH compris entre 7 et 8.

4. Procédé selon l'une quelconque des revendications précédentes, **caractérisé en ce que** ledit carbonate métallique séparé est traité avec de l'acide citrique ou de l'acide oxalique, afin d'obtenir un métal de valence zéro, dans lequel ledit métal de valence zéro est recyclé dans la deuxième étape de réaction (102).

5. Appareil (10) de capture et de conversion de dioxyde de carbone (CO₂) selon les revendications 1 à 4, comprenant :
un premier réacteur (11) ayant un premier module d'entrée (12) pour le dioxyde de carbone gazeux, un deuxième module d'entrée (13) pour une solution alcaline aqueuse comprenant un hydroxyde de métal alcalin, au moins un module de sortie (14) configuré pour dissoudre le dioxyde de carbone gazeux dans la solution alcaline aqueuse comprenant un hydroxyde de métal alcalin, de telle sorte que le dioxyde de carbone réagit avec l'hydroxyde de métal alcalin pour former un bicarbonate,
**caractérisé en ce que**
l'appareil comprend un deuxième réacteur (21) ayant un module d'entrée (22) pour la solution de bicarbonate, au moins un module de sortie (23), dans lequel ledit deuxième réacteur (21) est relié de manière opérationnelle audit premier réacteur (11) et est configuré pour mettre en contact la solution de bicarbonate avec une espèce métallique à valence zéro dans des conditions anaérobies pour former un mélange réactionnel de H₂, de carbonate métallique solide et d'une solution alcaline aqueuse
**et que** ledit deuxième réacteur (21) comprend un module de séparation liquide/solide (24) configuré pour séparer le mélange réactionnel en une solution alcaline aqueuse régénérée et un carbonate métallique solide, ladite solution alcaline aqueuse régénérée étant recyclée dans le premier réacteur (11).

6. Appareil selon la revendication 5 comprenant un troisième réacteur (31) ayant un module d'entrée (32) et au moins un module de sortie (33), ledit troisième réacteur (31) étant configuré pour recevoir du carbonate de Fe provenant du deuxième réacteur (21), mettre en contact ledit carbonate de Fe avec de l'acide citrique ou de l'acide oxalique pour former du Fe avec valence zéro, dans lequel ledit Fe avec valence zéro est recyclé dans le deuxième réacteur (21).
